(19) **Europäisches Patentamt**

**European Patent Office**

**Office européen des brevets**

(11) **EP 0 634 387 B1**

(12) **EUROPÄISCHE PATENTSCHRIFT**

(45) Veröffentlichungstag und Bekanntmachung des Hinweises auf die Patenterteilung:
**22.10.1997 Patentblatt 1997/43**

(51) Int. Cl.$^6$: **C07C 68/00**, C07C 69/96

(21) Anmeldenummer: **94110392.1**

(22) Anmeldetag: **04.07.1994**

(54) **Verfahren zur Herstellung von Dimethylcarbonat**

Process for the preparation of dimethyle carbonate

Procédé de préparation du carbonate de diméthyle

(84) Benannte Vertragsstaaten:
**BE DE ES FR GB IT NL**

(30) Priorität: **15.07.1993 DE 4323680**
**15.07.1993 DE 4323678**
**15.07.1993 DE 4323679**

(43) Veröffentlichungstag der Anmeldung:
**18.01.1995 Patentblatt 1995/03**

(73) Patentinhaber: **BAYER AG**
**51368 Leverkusen (DE)**

(72) Erfinder:
• **Jentsch, Joerg-Dietrich, Dr.**
**D-45468 Mülheim (DE)**

• **Klausener, Alexander, Dr.**
**D-50670 Köln (DE)**
• **Landscheidt, Heinz, Dr.**
**D-47057 Duisburg (DE)**
• **Lenders, Bernd, Dr.**
**D-47800 Krefeld (DE)**
• **Pennemann, Bernd, Dr.**
**D-51061 Köln (DE)**
• **Wolters, Erich, Dr.**
**D-50939 Köln (DE)**
• **Zirngiebl, Eberhard, Dr.**
**D-51061 Köln (DE)**

(56) Entgegenhaltungen:
**EP-A- 0 503 091          EP-A- 0 503 618**
**EP-A- 0 523 508**

**Beschreibung**

Die vorliegende Erfindung betrifft ein Verfahren zur Herstellung von Dimethylcarbonat durch Umsetzung von Kohlenmonoxid mit Methylnitrit in Gegenwart eines Katalysators, der durch Aufbringung einer oder mehrerer Palladiumverbindungen mit auf das Pd gerichteten N-haltigen Liganden gegebenenfalls in Verbindung mit einem oder mehreren Promotoren aus der Reihe der Elemente Fe, Co, Ni, V, Nb, Mo, Ta, Ti, Cu, Cr, der Seltenen Erden und/oder deren Verbindungen und/oder einem oder mehreren Promotoren aus der Reihe der Alkali- und Erdalkalihalogenide auf ein geeignetes Trägermaterial hergestellt wurde.

Dialkylcarbonate sind von allgemeiner chemischer und technischer Bedeutung. So ist beispielsweise Diethylcarbonat ein ausgezeichnetes Lösungsmittel im mittleren Siedebereich. Des weiteren sind die Dialkylcarbonate ausgezeichnete Carbonylierungs-und Acylierungsreagenzien. Sie haben große Bedeutung bei der Herstellung von anderen Carbonaten, wie beispielsweise Diphenylcarbonat, von Urethanen und von Harnstoffen. Schließlich eignen sie sich aufgrund ihres hohen Sauerstoffgehalts als Treibstoffadditive zur Verbesserung der Klopffestigkeit von Ottokraftstoffen.

Es ist bekannt, daß Dialkylcarbonate durch Umsetzung von Phosgen bzw. von Chlorameisensäurealkylestern mit Alkoholen hergestellt werden können. Es besteht jedoch ein steigendes Interesse daran, den Einsatz des giftigen Phosgens bzw. der davon abgeleiteten Zwischenprodukte, wie Chlorameisensäureester, durch andere Verfahren abzulösen.

Hier sind insbesondere Verfahren wichtig, in denen Kohlenmonoxid in der Gasphase mit Alkylnitriten an heterogenen, Platinmetalle enthaltenden Katalysatoren umgesetzt wird. So wird in der Zeitschrift für Katalytische Forschung (China) Vol. 10(1) S. 75 bis 78 (März 1989) die Umsetzung von Kohlenmonoxid mit Methylnitrit an einem $PdCl_2$-haltigen Aktivkohle-Trägerkatalysator beschrieben, wobei neben Dimethyloxalat überwiegend Dimethylcarbonat entsteht.

In DE-OS 41 23 603 wird durch Anwendung eines Palladiumchlorid-Katalysators mit $\gamma$-$Al_2O_3$ als Trägermaterial eine hohe Selektivität, sowohl bezogen auf Kohlenmonoxid als auch auf Methylnitrit, bei gleichzeitig hohem Umsatz erzielt. Allerdings muß zur Aufrechterhaltung der katalytischen Aktivität dem Eduktgemisch Chlorwasserstoffgas in Mengen bis zu 1000 ppm (Volumen) zugesetzt werden.

In EP 503 618 wird die Herstellung von Dialkylcarbonaten auf ähnliche Weise unter Verwendung eines Katalysators beschrieben, der ein Platinmetall, mindestens ein Element aus der Reihe Fe, Cu, Bi, Co, Ni oder Sn, mindestens einen weiteren Zusatz aus der Reihe V, Mo oder W sowie mindestens ein Halogenid enthält. Nachteilig an diesem Verfahren ist die Tatsache, daß nur geringe Umsätze an Methylnitrit erzielt werden. Desweiteren wird eine zum Teil erhebliche Desaktivierung der beschriebenen Katalysatoren bereits nach wenigen Stunden beobachtet. Dies bedeutet für eine technische Ausführung des Verfahrens einen erheblichen Mehraufwand, der für Katalysatorregenerierung bzw. Katalysatorwechsel betrieben werden muß. In EP 503 091 wird für den gleichen Zweck die Verwendung eines ähnlichen Katalysators beschrieben, der sich von dem in EP 503 618 dadurch unterscheidet, daß an Stelle von V, Mo bzw. W auch $H_2SO_4$ oder $H_3PO_4$ treten kann und daß keine (Erd)Alkalimetallverbindungen vorliegen. Sowohl in EP '618 als auch EP '091 werden Molybdat und Vanadat in den Erfindungs- und Vergleichsbeispielen gleichermaßen in Form ihrer Ammoniumsalze eingesetzt; $NH_4^{\oplus}$ bzw. $NH_3$ tragen demnach zur beanspruchten Erfindung in EP '618 und EP '091 nicht bei.

In der vorliegenden Erfindung konnten diese Nachteile durch Einsatz des erfindungsgemäßen Katalysators überraschenderweise überwunden werden.

Es wurde ein Verfahren zur kontinuierlichen Herstellung von Dimethylcarbonat durch Umsetzung von Kohlenmonoxid mit Methylnitrit in Anwesenheit eines Inertgases, in Anwesenheit von Methanol sowie in Anwesenheit oder Abwesenheit von Stickstoffmonoxid an einem Palladium-Trägerkatalysator bei erhöhter Temperatur in kontinuierlicher Gasphasenreaktion gefunden, das dadurch gekennzeichnet ist, daß bei einem Volumenverhältnis von Methylnitrit : Kohlenmonoxid von 0,1 : 1 bis 10 : 1, einem Druck von 0,5 bis 6 bar und einer Temperatur von 50 - 200°C gearbeitet wird, wobei der Katalysator durch Aufbringung einer oder mehrerer Palladiumverbindungen der allgemeinen Formel

$$[Pd(N(R^1,R^2,R^3))_n]X^1_2 \qquad\qquad (I),$$

in der

$R^1$, $R^2$ und $R^3$ unabhängig voneinander Wasserstoff, $C_1$-$C_4$-Alkyl, Phenyl oder $C_7$-$C_{10}$-Aralkyl bedeuten und $R^3$ zusätzlich -$A^1$-$N(R^4,R^5)$ bedeuten kann, worin -$A^1$- geradkettiges oder verzweigtes $C_1$-$C_8$-Alkylen, durch -O- oder -$NR^6$- unterbrochenes $C_2$-$C_8$-Alkylen oder Phenylen darstellt und wobei $R^2$ und $R^3$ bzw. $R^4$ und $R^5$ gemeinsam mit dem N-Atom, das sie substituierten, das Imidazolin-, Piperidin- oder Morpholinsystem bilden können und weiterhin $R^4$, $R^5$ und $R^6$ unabhängig voneinander Wasserstoff oder $C_1$-$C_4$-Alkyl bedeuten und $R^1$, $R^2$ und $R^3$ bzw. $R^2$, $R^3$ und $A^1$ bzw. $A^1$, $R^4$ und $R^5$ jeweils gemeinsam und zusammen mit dem N-Atom, das sie substituieren, das Pyridin- oder Chinolinsystem darstellen können, mit der Maßgabe, daß $R^1$, $R^2$ und $R^3$ nicht gleichzeitig Wasserstoff bedeuten,

n die Gesamtzahl der auf das Pd gerichteten N-Atome in den Liganden angibt und 2, 3 oder 4 bedeutet und

$X^1$ ein Äquivalent Fluorid, Chlorid, Bromid, Cyanid, Nitrat, Sulfat, Phosphat oder $C_1$-$C_6$-Carboxylat ist,

gegebenenfalls in Verbindung mit einem oder mehreren Promotoren aus der Reihe der Elemente Fe, Co, Ni, V, Nb, Mo, Ta, Ti, Cu, Cr, der Seltenen Erden und/oder deren Verbindungen und/oder gegebenenfalls mit einem oder mehreren Promotoren aus der Reihe der Alkali- und Erdalkalihalogenide auf ein geeignetes Trägermaterial hergestellt wurde.

In bevorzugter Weise wird eine Palladiumverbindung der Formel

$$[Pd(N(R^{11},R^{12},R^{13}))_n]X^2_2 \qquad (II)$$

eingesetzt, in der

$R^{11}, R^{12}$ und $R^{13}$ unabhängig voneinander Wasserstoff, $C_1$-$C_4$-Alkyl, bevorzugt $C_1$-$C_2$-Alkyl, besonders bevorzugt Methyl bedeuten, wobei $R^{13}$ zusätzlich Phenyl, Benzyl oder -$A^2$-N($R^{14}$,$R^{15}$) bedeuten kann, worin -$A^2$- geradkettiges oder verzweigtes $C_2$-$C_6$-Alkylen, bevorzugt Dimethylen oder Trimethylen, bedeutet und wobei weiterhin $R^{12}$ und $R^{13}$ und unabhängig davon $R^{14}$ und $R^{15}$ gemeinsam mit dem N-Atom, das sie substituieren, das Imidazolin-, Piperidin- oder Morpholinsystem bilden können, mit der Maßgabe, daß $R^{11}$, $R^{12}$ und $R^{13}$ nicht gleichzeitig Wasserstoff bedeuten,

$X^2$ ein Äquivalent Fluorid, Chlorid, Bromid oder Sulfat ist und

n die oben angegebene Bedeutung hat.

$C_1$-$C_4$-Alkyl ist beispielsweise Methyl, Ethyl, Propyl, Isopropyl, Butyl, Isobutyl, bevorzugt Methyl oder Ethyl. $C_7$-$C_{10}$-Aralkyl ist beispielsweise Benzyl, Phenylethyl ($\alpha$ oder $\beta$), Phenyl-propyl oder Phenyl-butyl, bevorzugt Benzyl. $C_1$-$C_8$-Alkylen oder durch -O- oder -$NR^6$- unterbrochenes $C_2$-$C_8$-Alkylen ist beispielsweise Methylen, Dimethylen, Trimethylen, Tetramethylen, Hexamethylen, Octamethylen, 1-Methyl-dimethylen, 1- oder 2-Methyl-trimethylen, -tetramethylen oder -pentamethylen, 2,3-Butylen oder analoges Alkylen, bevorzugt solches mit 2-6 C-Atomen; -$C_2H_4$-O-$C_2H_4$-, -$C_2H_4$-O-$C_2H_4$-O-$C_2H_4$-, $C_2H_4$-NH-$C_2H_4$-, -$C_2H_4$-N($CH_3$)-$C_2H_4$- und analoge Gruppen.

In der angegebenen Art können auch heterocyclische, N-haltige Ringsysteme gebildet werden, deren N-Atome eine Ligandenbindung zum Pd ausbilden.

Für das erfindungsgemäße Verfahren eignen sich alle dem Fachmann bekannten Trägermaterialien, wie beispielsweise Aktivkohle, Zeolithe, Alumosilikate, Metallphosphate, Oxide, Hydroxide und Oxidhydrate des Aluminiums und des Siliciums, Kieselgure und Kieselsäuren, Molekularsiebe, Diatomeenerden, Montmorillonite, Schichtsilikate, Oxide des Titans, Zinks, Eisens und Mangens oder Heteropolysäuren.

Der erfindungsgemäße Katalysator enthält mindestens einen, gegebenenfalls aber auch mehrere Promotoren aus der Reihe der Elemente Fe, Co, Ni, V, Nb, Mo, Ta, Ti, Cu, Cr, der Seltenen Erden und/oder deren Verbindungen und/oder mindestens einen oder mehrere Promotoren aus der Reihe der Alkali- und Erdalkalihalogenide in einer Gesamtmenge von 0,1 bis 4 Gew.-%, bezogen auf das Gesamtgewicht des Katalysators.

Die Herstellung der erfindungsgemäßen Katalysatoren kann beispielsweise dadurch erfolgen, daß Palladiumhalogenide mit Alkali- oder Erdalkalimetallhalogeniden in einem geeigneten Lösungsmittel wie Wasser, Methanol oder Aceton in Lösung gebracht werden und die so erhaltenen Lösungen anschließend mit Stickstoffverbindungen der allgemeinen Formel

$$N(R^1R^2R^3) \qquad (III),$$

in der

$R^1$, $R^2$ und $R^3$ die angegebene Bedeutung haben,

oder mit deren Salzen umgesetzt werden.

Die Aufbringung der erhaltenen Lösungen auf den jeweiligen Katalysatorträger erfolgt dann in einer dem Fachmann bekannten Art, beispielsweise durch Tränkung, durch Absorption oder durch Besprühen.

Dabei können die bevorzugt in Form ihrer Verbindungen zum Einsatz kommenden Promotoren aus der Reihe der Elemente Fe, Co, Ni, V, Nb, Mo, Ta, Ti, Cu, Cr und der Seltenen Erden und/oder die Promotoren aus der Reihe der Alkali-und Erdalkalihalogenide entweder gemeinsam mit den genannten Palladiumverbindungen oder aber getrennt von ihnen auf die genannten Träger aufgebracht werden. Bevorzugte Promotoren aus der Reihe der Elemente sind Fe, Co, Ni, Cu, V und/oder Mo, besonders bevorzugt Cu, Fe, V und/oder Mo bzw. deren Verbindungen. Bevorzugt unter den (Erd)Alkalihalogeniden sind Fluoride oder Chloride von Li, Na, K, Cs, Be, Mg und/oder Ca, besonders bevorzugt von Li, Na und/oder K.

Eine andere Möglichkeit, den erfindungsgemäßen Katalysator herzustellen, besteht darin, den Katalysator mit einer Palladiumhalogenid-Lösung zu tränken, die gegebenenfalls einen oder mehrere der genannten Promotoren bzw. ihrer Verbindungen enthält, und das so gewonnene Produkt entweder mit einer Lösung des betreffenden Amins (II) oder mit gasförmigem Amin (II) zu behandeln.

Die dem erfindungsgemäßen Verfahren zugrundeliegende Reaktion läuft im Sinne der nachfolgenden Reaktionsgleichung ab:

$$CO + 2\,MeONO \rightarrow O{=}C(OMe)_2 + 2\,NO$$

Während es grundsätzlich möglich ist, Kohlenmonoxid mit Methylnitrit in Abwesenheit anderer gasförmiger Reaktionskomponenten oder Reaktionshilfsstoffe umzusetzen, beispielsweise dann, wenn man mit außerhalb der Explosionsgrenzen liegenden Gemischzusammensetzungen arbeitet, wird erfindungsgemäß ein Inertgas zur Verdünnung der Reaktionspartner herangezogen. Als Inertgase kommen dazu beispielsweise Edelgase, Stickstoff und Kohlendioxid, bevorzugt Argon, Stickstoff oder Kohlendioxid, besonders bevorzugt Stickstoff und Kohlendioxid in Frage.

Die Menge des Inertgases beträgt 20 bis 85 Vol.-%, bezogen auf das gesamte in den Reaktor einzuführende Gasvolumen. Das Inertgas und gegebenenfalls darin enthaltene nicht umgesetzte Restmengen der Reaktionspartner können recycliert werden.

Das Volumenverhältnis der Reaktionspartner Methylnitrit und Kohlenmonoxid zueinander beträgt 0,1:1 bis 10:1, bevorzugt 0,2:1 bis 4:1, besonders bevorzugt 0,3:1 bis 3:1.

Das umzusetzende Gasgemisch kann weiterhin geringe Mengen an Methanol, sowie geringe Mengen an Stickstoffmonoxid enthalten. Methanol bzw. Stickstoffmonoxid liegen unabhängig voneinander jeweils beispielsweise in einer Menge von 0 bis 10 Vol.-%, beides bezogen auf das Gesamtvolumen des einzusetzenden Gasgemisches, vor. Solche Zusätze an Methanol bzw. Stickstoffmonoxid können etwa aus der Herstellung des Methylnitrits entstammen und beispielsweise mit diesem in das Reaktionsgasgemisch hineingetragen werden.

Dem umzusetzenden Gasgemisch kann weiterhin als Reaktionshilfsstoff eine geringe Menge eines Aktivators zugesetzt werden. Dabei handelt es sich um Halogene oder Halogenwasserstoffe wie Chlor, Brom, Chlorwasserstoff und Bromwasserstoff, die in einer Menge von 0 bis 2000 ppm, bevorzugt 0 bis 1000 ppm, besonders bevorzugt 0 bis 750 ppm (jeweils Volumen) zudosiert werden. Bevorzugt wird Chlor oder Chlorwasserstoff zugesetzt.

Das erfindungsgmäße Verfahren wird bei einer Temperatur von 50-200°C, bevorzugt 70-170°C, besonders bevorzugt 75-150°C und bei einem Druck von 0,5-6 bar, bevorzugt 1-6 bar, besonders bevorzugt 1,5-4,5 bar durchgeführt.

### Beispiele

### Definitionen

Die Raumzeitausbeute (Space Time Yield STY) in [g/(l*h)] für Dimethylcarbonat in dem angegebenen Beispiel berechnet sich nach

$$STY = \frac{m_{DMC}}{V_{Kat}*t}\,[g/(l*h)]\,,$$

wobei $m_{DMC}$ die Menge des gebildeten Dimethylcarbonats (DMC), $V_{Kat}$ das Katalysatorvolumen und $t$ die Zeit bedeuten.

Die Selektivität S (%) der Bildung des DMC, bezogen auf CO, wird berechnet nach

$$S = \frac{n_{DMC}}{n_{DMC} + 2 * n_{ODME} + n_{AME} + n_{FDA}} * 100\ (\%)$$

wobei

$n_{DMC}$ = Stoffmenge Dimethylcarbonat
$n_{ODME}$ = Stoffmenge Oxalsäuredimethylester
$n_{AME}$ = Stoffmenge Ameisensäuremethylester
$n_{FDA}$ = Stoffmenge Formaldehyddimethylacetal

bedeuten.

### Katalysatorherstellung

0,835 g $PdCl_2$ wurden unter Zusatz von 0,6 g Natriumchlorid in 29 ml Wasser gelöst. Bei Raumtemperatur werden unter Rühren 0,6 g Ethylendiamin hinzugefügt.

100 ml Aktivkohle Norit ROX 0,8 wurden mit dieser Lösung getränkt und anschließend im Stickstoffstrom getrocknet.

### Verfahrensbeschreibung

In einem vertikal aufgestellten Rohrreaktor (Glas, Länge 50 cm, Durchmesser 4 cm) wurden zwischen einer Füllung aus Raschig-Ringen 20 ml des Katalysators eingebracht.

Das Glasrohr wurde auf 120°C erhitzt und ein Gasgemisch aus 50 Vol.-% Stickstoff 30 Vol.-% Methylnitrit, 15 Vol.-% Kohlenmonoxid und 5 Vol.-% Methanol mit einer Gesamtgasbelastung von 5000 l/h wurde hindurchgeleitet.

Das dem Reaktor entströmende Gas wurde auf 5°C gekühlt und die erhaltene kondensierte Phase mittels Gaschromatographie untersucht.

Die nicht kondensierten Produkte wurden mittels IR-Spektroskopie und Massenspektroskopie erfaßt. Dimethylcarbonat wurde nach 4 h mit einer Raum-Zeit-Ausbeute von STY = 190,1 g/(l*h) und einer Selektivität von S = 97,6 % gebildet. Nach 30 h betrugen die Raum-Zeit-Ausbeute STY = 212,9 g/(l*h) und die Selektivität S = 88,4 %.

## Patentansprüche

1. Verfahren zur kontinuierlichen Herstellung von Dimethylcarbonat durch Umsetzung von Kohlenmonoxid (CO) mit Methylnitrit in Anwesenheit eines Inertgases, in Anwesenheit von Methanol sowie in Anwesenheit oder Abwesenheit von Stickstoffmonoxid an einem Palladium-Trägerkatalysator bei erhöhter Temperatur in der Gasphase, dadurch gekennzeichnet, daß bei einem Volumenverhältnis von Methylnitrit:Kohlenmonoxid von 0,1:1 bis 10:1, einem Druck von 0,5 bis 6 bar und einer Temperatur von 50-200°C gearbeitet wird, wobei der Katalysator durch Aufbringung einer oder mehrerer Palladium-Verbindungen der allgemeinen Formel

$$[Pd(N(R^1,R^2,R^3))_n]X^1_2 \hspace{3cm} (I),$$

in der

$R^1, R^2$ und $R^3$ unabhängig voneinander Wasserstoff $C_1$-$C_4$-Alkyl, Phenyl oder $C_7$-$C_{10}$-Aralkyl bedeutet und $R^3$ zusätzlich -$A^1$-$N(R^4,R^5)$ bedeuten kann, worin -$A^1$- geradkettiges oder verzweigtes $C_1$-$C_8$-Alkylen, durch -O- oder -$NR^6$-unterbrochenes $C_2$-$C_8$-Alkylen oder Phenylen darstellt und wobei $R^2$ und $R^3$ bzw. $R^4$ und $R^5$ jeweils gemeinsam mit dem N-Atom, das sie substituieren, das Imidazolin-, Piperidin- oder Morpholinsystem bilden können und weiterhin $R^4$, $R^5$ und $R^6$ unabhängig voneinander Wasserstoff oder $C_1$-$C_4$-Alkyl bedeuten und $R^1$, $R^2$ und $R^3$ bzw. $R^2$, $R^3$ und $A^1$ bzw. $A^1$, $R^4$ und $R^5$ jeweils gemeinsam und zusammen mit dem N-Atom, das sie substituieren, das Pyridin- oder Chinolinsystem darstellen können, mit der Maßgabe, daß $R^1$, $R^2$ und $R^3$ nicht gleichzeitig Wasserstoff bedeuten,

n die Gesamtzahl der auf das Pd gerichteten N-Atome in den Liganden angibt und 2, 3 oder 4 bedeutet und

$X^1$ ein Äquivalent Fluorid, Chlorid, Bromid, Cyanid, Nitrat, Sulfat, Phosphat oder $C_1$-$C_6$-Carboxylat ist,

gegebenenfalls in Verbindung mit einem oder mehreren Promotoren aus der Reihe der Elemente Fe, Co, Ni, V, Nb, Mo, Ta, Ti, Cu, Cr, der Seltenen Erden und/oder deren Verbindungen und/oder gegebenenfalls mit einem oder mehreren Promotoren aus der Reihe der Alkali- und Erdalkalihalogenide auf ein geeignetes Trägermaterial hergestellt wurde.

2. Verfahren nach Anspruch 1, dadurch gekennzeichnet, daß das Trägermaterial aus der Reihe der Aktivkohlen, Zeolithe, Alumosilikate, der Metallphosphate, der Oxide, Hydroxide und Oxyhydrate des Aluminiums und Siliciums, der Kieselgure und Kieselsäuren, der Molekularsiebe, der Diatomeenerden, Montmorillonite, Schichtsilikate, der Oxide des Titans, Zinks, Eisens und Mangans oder der Heteropolysäuren stammt.

3. Verfahren nach Anspruch 1, dadurch gekennzeichnet, daß eine Palladiumverbindung der Formel

$$[Pd(N(R^{11},R^{12},R^{13}))_n]X^2_2 \qquad \text{(II)}$$

eingesetzt wird, in der

$R^{11}, R^{12}$ und $R^{13}$ unabhängig voneinander Wasserstoff, $C_1$-$C_4$-Alkyl, bevorzugt $C_1$-$C_2$-Alkyl, besonders bevorzugt Methyl bedeuten, wobei $R^{13}$ zusätzlich Phenyl, Benzyl oder -$A^2$-$N(R^{14},R^{15})$ bedeuten kann, worin -$A^2$- geradkettiges oder verzweigtes $C_2$-$C_6$-Alkylen, bevorzugt Dimethylen oder Trimethylen, bedeutet und wobei weiterhin $R^{12}$ und $R^{13}$ und unabhängig davon $R^{14}$ und $R^{15}$ gemeinsam mit dem N-Atom, das sie substituieren, das Imidazolin-, Piperidin- oder Morpholinsystem bilden können, mit der Maßgabe, daß $R^{11}$, $R^{12}$ und $R^{13}$ nicht gleichzeitig Wasserstoff bedeuten,

$X^2$ ein Äquivalent Fluorid, Chlorid, Bromid oder Sulfat ist und

n die in Anspruch 1 gegebene Bedeutung hat.

4. Verfahren nach Ansprüchen 1 und 2, dadurch gekennzeichnet, daß das Trägermaterial der Reihe der Aktivkohlen, Zeolithe, Alumosilikate, der Metallphosphate, der Aluminiumoxide, der Manganoxide oder der Heteropolysäuren entstammt und bevorzugt Aktivkohle oder ein Aluminiumoxid ist.

5. Verfahren nach Ansprüchen 1 bis 4, dadurch gekennzeichnet, daß ein oder mehrere Promotoren aus der Reihe der Elemente Fe, Co, Ni, Cu, V und Mo und/oder deren Verbindungen und/oder Fluoride oder Chloride der Alkali- und Erdalkalimetalle Li, Na, K, Cs, Be, Mg und Ca als Bestandteile des Katalysators Verwendung finden.

6. Verfahren nach Anspruch 5, dadurch gekennzeichnet, daß Promotoren aus der Reihe der Elemente Cu, Fe, V und Mo und/oder deren Verbindungen und/oder Fluoride oder Chloride der Elemente Li, Na und K als Bestandteile des Katalysators Verwendung finden.

7. Verfahren nach Ansprüchen 1 bis 6, dadurch gekennzeichnet, daß ein Inertgas aus der Gruppe der Edelgase, des Stickstoffs und des Kohlendioxids, bevorzugt Argon, Stickstoff oder Kohlendioxid, besonders bevorzugt Stickstoff oder Kohlendioxid, zum Einsatz kommt.

8. Verfahren nach Ansprüchen 1 bis 7, dadurch gekennzeichnet, daß als weiterer gegebenenfalls zugesetzter und als Aktivator wirkender Reaktionshilfsstoff Chlor, Brom, Chlorwasserstoff oder Bromwasserstoff, bevorzugt Chlor oder Chlorwasserstoff in einer Menge von 0-2000 ppm, bevorzugt 0-1000 ppm, besonders bevorzugt 0-750 ppm (Volumen) verwendet wird.

9. Verfahren nach Ansprüchen 1 bis 8, dadurch gekennzeichnet, daß in einem Bereich von 70 bis 170°C für die Reaktionstemperatur 1 bis 6 bar für den Reaktionsdruck, bevorzugt bei 75 bis 150°C und 1,5 bis 4,5 bar, gearbeitet wird.

10. Verfahren nach Ansprüchen 1 bis 9, dadurch gekennzeichnet, daß die Volumenanteile im Reaktandengasgemisch für Methylnitrit 5 % bis 50 %, bevorzugt 15 % bis 45 %, besonders bevorzugt 30 % bis 35 %, für Kohlenmonoxid 10 % bis 30 %, bevorzugt 10 % bis 25 %, besonders bevorzugt 10 % bis 20 % und für das zugesetzte Inertgas 20 % bis 85 %, bevorzugt 45 % bis 70 %, besonders bevorzugt 50 % bis 65 % betragen.

**Claims**

1. Process for the continuous preparation of dimethyl carbonate by reacting carbon monoxide (CO) with methyl nitrite in the presence of an inert gas, in the presence of methanol and in the presence or absence of nitric oxide on a supported palladium catalyst at elevated temperature in the gas phase, characterized in that it is carried out at a volume ratio methyl nitrite:carbon monoxide of 0.1:1 to 10:1, a pressure of 0.5 to 6 bar and a temperature of 50-200°C, the catalyst having been prepared by the application to a suitable support of one or more palladium compounds of the general formula

$$[Pd(N(R^1,R^2,R^3))_n]X^1_2 \qquad \text{(I)},$$

in which

$R^1$, $R^2$ and $R^3$ independently of one another are hydrogen, $C_1$-$C_4$-alkyl, phenyl or $C_7$-$C_{10}$-aralkyl and $R^3$ can

additionally be $-A^1-N(R^4,R^5)$, wherein $-A^1-$ is linear or branched $C_1-C_8$-alkylene, $C_2-C_8$-alkylene interrupted by -O- or $-NR^6-$, or phenylene, it being possible for $R^2$ and $R^3$ or $R^4$ and $R^5$, in each case together with the N atom on which they are substituents, to form the imidazoline, piperidine or morpholine system, and $R^4$, $R^5$ and $R^6$ independently of one another being hydrogen or $C_1-C_4$-alkyl, and $R^1$, $R^2$ and $R^3$, or $R^2$, $R^3$ and $A^1$ or $A^1$, $R^4$ and $R^5$, in each case together with the N atom on which they are substituents, can be the pyridine or quinoline system, with the proviso that $R^1$, $R^2$ and $R^3$ are not simultaneously hydrogen,

n      indicates the total number of Pd-oriented N atoms in the ligands and is 2, 3 or 4, and

$X^1$      is one equivalent of fluoride, chloride, bromide, cyanide, nitrate, sulphate, phosphate or $C_1-C_6$-carboxylate,

optionally in combination with one or more promoters from the group consisting of the elements Fe, Co, Ni, V, Nb, Mo, Ta, Ti, Cu and Cr, the rare earths and/or compounds thereof, and/or optionally with one or more promoters from the group consisting of alkali metal and alkaline earth metal halides.

2. Process according to Claim 1, characterized in that the support is taken from the group consisting of activated charcoals, zeolites, aluminosilicates, metal phosphates, oxides, hydroxides and hydrated oxides of aluminium and silicon, kieselguhrs and silicic acids, molecular sieves, diatomaceous earths, montmorillonites, phyllosilicates, oxides of titanium, zinc, iron and manganese, and heteropolyacids.

3. Process according to Claim 1, characterized in that a palladium compound of the formula

$$[Pd(N(R^{11},R^{12},R^{13}))_n]X^2_2 \qquad\qquad (II)$$

is used in which

$R^{11}$, $R^{12}$ and $R^{13}$      independently of one another are hydrogen or $C_1-C_4$-alkyl, preferably $C_1-C_2$-alkyl and particularly preferably methyl, it being possible for $R^{13}$ additionally to be phenyl, benzyl or $-A^2-N(R^{14},R^{15})$, wherein $-A^2-$is linear or branched $C_2-C_6$-alkylene, preferably dimethylene or trimethylene, and it also being possible for $R^{12}$ and $R^{13}$ and independently thereof $R^{14}$ and $R^{15}$, together with the N atom on which they are substituents, to form the imidazoline, piperidine or morpholine system, with the proviso that $R^{11}$, $R^{12}$ and $R^{13}$ are not simultaneously hydrogen,

$X^2$      is one equivalent of fluoride, chloride, bromide or sulphate, and

n      is as defined in Claim 1.

4. Process according to Claims 1 and 2, characterized in that the support is taken from the group consisting of activated charcoals, zeolites, aluminosilicates, metal phosphates, aluminium oxides, manganese oxides and heteropolyacids and is preferably activated charcoal or an aluminium oxide.

5. Process according to Claims 1 to 4, characterized in that one or more promoters from the group consisting of the elements Fe, Co, Ni, Cu, V and Mo and/or compounds thereof, and/or the fluorides or chlorides of the alkali metals and alkaline earth metals Li, Na, K, Cs, Be, Mg and Ca, are used as constituents of the catalyst.

6. Process according to Claim 5, characterized in that promoters from the group consisting of the elements Cu, Fe, V and Mo and/or compounds thereof, and/or the fluorides or chlorides of the elements Li, Na and K, are used as constituents of the catalyst.

7. Process according to Claims 1 to 6, characterized in that an inert gas from the group consisting of noble gases, nitrogen and carbon dioxide, preferably argon, nitrogen and carbon dioxide and particularly preferably nitrogen and carbon dioxide, is used.

8. Process according to Claims 1 to 7, characterized in that chlorine, bromine, hydrogen chloride or hydrogen bromide, preferably chlorine or hydrogen chloride, is optionally added as a reaction aid serving as an activator, in an amount of 0-2000 ppm, preferably 0-1000 ppm and particularly preferably 0-750 ppm (by volume).

**9.** Process according to Claims 1 to 8, characterized in that the reaction is carried out in the temperature range 70 to 170°C and the pressure range 1 to 6 bar, preferably at 75 to 150°C and 1.5 to 4.5 bar.

**10.** Process according to Claims 1 to 9, characterized in that the proportions by volume in the reactant gas mixture are 5% to 50%, preferably 15% to 45% and particularly preferably 30% to 35% of methyl nitrite, 10% to 30%, preferably 10% to 25% and particularly preferably 10% to 20% of carbon monoxide and 20% to 85%, preferably 45% to 70% and particularly preferably 50% to 65% of added inert gas.

**Revendications**

**1.** Procédé de préparation continue de carbonate de diméthyle par réaction du monoxyde de carbone (CO) avec le nitrite de méthyle en présence d'un gaz inerte, en présence de méthanol et en présence ou en l'absence de monoxyde d'azote sur un catalyseur palladium-support à haute température en phase gazeuse, caractérisé en ce que l'on opère à un rapport volumique nitrite de méthyle:monoxyde de carbone de 0,1:1 à 10:1, à une pression de 0,5 à 6 bar et à une température de 50-200°C, le catalyseur ayant été préparé par application sur un matériau support approprié d'un ou plusieurs composés du palladium de formule générale :

$$[Pd(N(R^1,R^2,R^3))_n]X^1{}_2 \tag{I}$$

dans laquelle

R$^1$, R$^2$ et R$^3$   représentent indépendamment les uns des autres l'hydrogène, alkyle en $C_1$-$C_4$, phényle ou aralkyle en $C_7$-$C_{10}$ et R$^3$ peut représenter en outre -A$^1$-N(R$^4$,R$^5$), où -A$^1$- représente un alkylène en $C_1$-$C_8$ linéaire ou ramifié, un alkylène en $C_2$-$C_8$ interrompu par -O- ou -NR$^6$- ou un phénylène et dans laquelle R$^2$ et R$^3$ ou R$^4$ et R$^5$ peuvent former dans chaque cas avec l'atome N qu'ils substituent le système de l'imidazoline, de la pipéridine ou de la morpholine et en outre R$^4$, R$^5$ et R$^6$ représentent indépendamment les uns des autres l'hydrogène ou alkyle en $C_1$-$C_4$ et R$^1$, R$^2$ et R$^3$ ou R$^2$, R$^3$ et A$^1$ ou A$^1$, R$^4$ et R$^5$ peuvent représenter dans chaque cas collectivement et avec l'atome N qu'ils substituent le système de la pyridine ou de la quinoléine, à condition que R$^1$, R$^2$ et R$^3$ ne représentent pas simultanément l'hydrogène,

n   indique le nombre total des atomes N dirigés vers Pd dans les ligands et représente 2, 3 ou 4 et

X$^1$   est un équivalent de fluorure, de chlorure, de bromure, de cyanure, de nitrate, de sulfate, de phosphate ou de carboxylate en $C_1$-$C_6$,

éventuellement en combinaison avec un ou plusieurs promoteurs de la série des éléments Fe, Co, Ni, V, Nb, Mo, Ta, Ti, Cu, Cr, des terres rares et/ou de leurs composés et/ou éventuellement avec un ou plusieurs promoteurs de la série des halogénures alcalins et alcalino-terreux.

**2.** Procédé selon la revendication 1, caractérisé en ce que le matériau support est issu de la série des charbons actifs, des zéolites, des aluminosilicates, des phosphates métalliques, des oxydes, hydroxydes ou oxydes hydratés de l'aluminium et du silicium, des kieselguhrs et des acides siliciques, des tamis moléculaires, des terres de diatomées, des montmorillonites, des silicates feuilletés, des oxydes du titane, du zinc, du fer et du manganèse ou des hétéropolyacides.

**3.** Procédé selon la revendication 1, caractérisé en ce que l'on utilise un composé du palladium de formule

$$[Pd(N(R^{11},R^{12},R^{13}))_n]X^2{}_2 \tag{II}$$

dans laquelle

R$^{11}$, R$^{12}$ et R$^{13}$   représentent indépendamment les uns des autres l'hydrogène, alkyle en $C_1$-$C_4$, de préférence alkyle en $C_1$-$C_2$, de préférence encore méthyle, R$^{13}$ pouvant représenter en outre phényle, benzyle ou - -A$^2$-N(R$^{14}$,R$^{15}$), où -A$^2$- représente un alkylène en $C_2$-$C_6$ linéaire ou ramifié, de préférence le diméthylène ou le triméthylène, et où, en outre, R$^{12}$ et R$^{13}$ et indépendamment de ceux-ci R$^{14}$ et R$^{15}$ peuvent former avec l'atome N qu'ils substituent le système de l'imidazoline, de la pipéridine ou de la morpholine, à condition que R$^{11}$, R$^{12}$ et R$^{13}$ ne représentent pas simultanément l'hydrogène,

X$^2$   est un équivalent de fluorure, de chlorure, de bromure ou de sulfate et

n   a la signification indiquée dans la revendication 1.

4. Procédé selon les revendications 1 et 2, caractérisé en ce que le matériau support est issu de la série des charbons actifs, des zéolites, des aluminosilicates, des phosphates métalliques, des oxydes d'aluminium, des oxydes de manganèse ou des hétéropolyacides et est de préférence un charbon actif ou un oxyde d'aluminium.

5. Procédé selon les revendications 1 à 4, caractérisé en ce qu'un ou plusieurs promoteurs de la série des éléments Fe, Co, Ni, Cu, V et Mo et/ou de leurs composés et/ou des fluorures ou chlorures des métaux alcalins et alcalino-terreux Li, Na, K, Cs, Be, Mg et Ca sont utilisés comme constituants du catalyseur.

6. Procédé selon la revendication 5, caractérisé en ce que des promoteurs de la série des éléments Cu, Fe, V et Mo et/ou de leurs composés et/ou des fluorures ou chlorures des éléments Li, Na et K sont utilisés comme constituants du catalyseur.

7. Procédé selon les revendications 1 à 6, caractérisé en ce qu'un gaz inerte du groupe des gaz nobles, de l'azote et du dioxyde de carbone, de préférence l'argon, l'azote ou le dioxyde de carbone, de préférence encore l'azote ou le dioxyde de carbone, est utilisé.

8. Procédé selon les revendications 1 à 7, caractérisé en ce que le chlore, le brome, le chlorure d'hydrogène ou le bromure d'hydrogène, de préférence le chlore ou le chlorure d'hydrogène, est utilisé comme adjuvant réactionnel supplémentaire éventuellement ajouté et agissant comme activateur, en une quantité de 0-2 000 ppm, de préférence de 0-1 000 ppm, de préférence encore de 0-750 ppm (en volume).

9. Procédé selon les revendications 1 à 8, caractérisé en ce que l'on opère dans un domaine de 70 à 170°C pour la température réactionnelle, de 1 à 6 bar pour la pression réactionnelle, de préférence entre 75 et 150°C et entre 1,5 et 4,5 bar.

10. Procédé selon les revendications 1 à 9, caractérisé en ce que les proportions volumiques dans le mélange gazeux des corps réagissants sont de 5 à 50 %, de préférence de 15 à 45 %, de préférence encore de 30 à 35 % pour le nitrite de méthyle, de 10 à 30 %, de préférence de 10 à 25 %, de préférence encore de 10 à 20 % pour le monoxyde de carbone et de 20 à 85 %, de préférence de 45 à 70 %, de préférence encore de 50 à 65 % pour le gaz inerte ajouté.